# EUROPEAN PATENT SPECIFICATION

(11) **EP 1 059 289 B1**
(45) Date of publication and mention of the grant of the patent: **27.07.2005**
(21) Application number: 00110990.9
(22) Date of filing: 29.05.2000
(51) Int. Cl.: C07C 331/32, C07C 333/10, C07C 333/26

(54) **Process for manufacture of N-alk(en)oxy(or aryloxy)carbonyl isothiocyanates and their derivatives in the presence of N,N-dialkylarylamine catalyst**
Verfahren zur Herstellung von N-Alk(en)oxy- oder N-Aryloxy-carbonyl-isothiocyanaten sowie deren Derivate in Anwesenheit eines N,N-Dialkyl-arylamins als Katalysator
Procédé de préparation de N-alkyl(ène)oxy ou N-aryloxy-carbonyl-isothiocyanates ainsi que leur dérivés en présence de N,N-dialkyl-arylamine comme catalyseur

(30) Priority: 10.06.1999 US 329405; 10.06.1999 US 329744
(43) Date of publication of application: 13.12.2000
(73) Proprietor: Bayer Corporation, Pittsburgh, PA 15205-9741 (US)
(72) Inventor: Kulkarni, Shekhar V., Shawnee, KS 66216 (US); Desai, Vijay, C., Shawnee, KS 66216 (US)
(74) Representative: Schwenk, Norbert, Dr.

(56) References cited:
- US-A- 4 659 853
- US-A- 4 778 921
- US-A- 6 066 754

## Description

### TECHNICAL FIELD OF THE INVENTION

The field of the present invention is the manufacture of carbonyl isothiocyanates and their derivatives. More particularly, the present invention pertains to an improved process for preparing N-alk(en)oxy(or aryloxy)carbonyl isothiocyanates and their derivatives, wherein the improvement comprises the presence of a N,N-dialk(en)ylarylamine as a catalyst in the reaction process.

### BACKGROUND OF THE INVENTION

Derivatives of carbonyl isothiocyanates are well known in the art, and various methods for their production are also known in the art. They can be used after further derivatization to prepare 5-alkoxy-substituted 1,2,4-triazolinon-3-ones which are usable as intermediates in the commercial preparation of certain agrochemicals.

U.S. Patent 4,659,853 discloses a process for producing derivatives of alkoxy, aryloxy and alkene isothiocyanates by reacting a haloformate, an alkali, alkaline earth metal, lead or ammonium thiocyanate and a compound having the formula R¹-Y-H wherein R¹ is an alkyl, aryl or alkoxy, Y is oxygen, sulfur or N-R², and R² is hydrogen or R¹; in the presence of a solvent or water and a catalyst. Suitable catalysts include pyridine, quinoline, pyrimidine, pyrazine, quinoxaline and the like.

U.S. Patent 4,778,921 describes a process for the preparation of alkoxy and aryloxy isothiocyanates which includes the reaction of a haloformate and an alkali or alkaline earth metal thiocyanate in the presence of water and a catalyst. The catalyst comprises a six- membered mononuclear or ten-membered fused polynuclear aromatic, heterocyclic compound having one or two nitrogen atoms as the only hetero atoms in the ring.

U.S. Patent 5,194,673 discloses a process for producing alkoxy and aryloxy isothiocyanates by the reaction of a haloformate and an alkali or alkaline earth metal thiocyanate in the presence of water and a catalyst. A co-catalyst is also used in the process to accelerate the reaction rate, increase product purity, and reduce the adverse effects of impurities in the thiocyanate reactants.

In the publication, Chem. Ber. 116, 2044, (1983), it is reported that the use of an aromatic heterocyclic nitrogen catalyst such as pyridine in carbon tetrachloride produced an alkoxythiocarbonyl isothiocyanate wherein the yield was only about 52%.

The most prevalent prior art methods comprise (i) the formation of the carbonyl isothiocyanate, (ii) the recovery and purification thereof, and (iii) the final reaction thereof with the appropriate co-reactant to produce the desired derivative. However, the known methods result in carbonyl isothiocyanates of low yield and purity. Thus, there is a need in the art for a process to produce carbonyl isothiocyanates and their derivatives in high yield and purity.

### BRIEF SUMMARY OF INVENTION

The present invention provides a process for the preparation of N-alk(en)oxy(or aryloxy)carbonyl isothiocyanate derivatives which includes reacting a haloformate compound of the general formula (I) 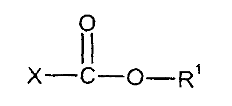 wherein R¹ represents a C₁ - C₈ alkyl radical, a C₂ - C₄ alkenyl radical, or a C₆ - C₁₀ aryl radical; and X represents a halogen atom;
with a thiocyanate of the general formula (II)

MSCN (II)

wherein M represents an alkali or alkaline earth metal, lead, or NH₄,
in the presence of an aqueous solvent or in the presence of an organic solvent, and in the presence of a catalytic amount of a N,N-dialk(en)ylarylamine of the general formula (III) 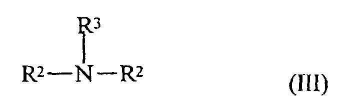 wherein the two R² each independently of another represent a C₁ - C₈ alkyl radical or a C₃ - C₆ alkenyl radical, or the two R² together represent a C₅ saturated heterocyclic ring or a C₄ saturated heterocylic ring wherein an oxygen atom may be part of the ring; and R³ represents an aryl group that can be a phenyl, a naphthyl, a substituted phenyl or a substituted naphthyl; to produce a N-alk(en)oxy(or aryloxy)carbonyl isothiocyanate intermediate product of the general formula (IV) 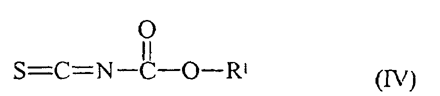 wherein R¹ is as defined above in formula (I); and reacting a compound of the general formula (V)

R⁴ - Y - H (V)

wherein R⁴ represents a C₁ - C₁₀ alkyl radical, a C₆ -C₁₀ aryl radical or a C₁ - C₈ alkoxy radical, and Y represents oxygen, sulfur or NR⁵, wherein R⁵ represents hydrogen or R⁴, with the intermediate product (IV) to produce a N-alk(en)oxy(or aryloxy)carbonyl isothiocyanate derivative of the general formula (VI) 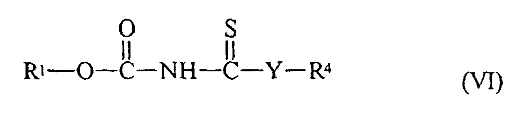 wherein R¹ is as defined above in formula (I), and R⁴ and Y are as defined above in formula (V).

The first reaction step for the preparation of the carbonyl isothiocyanates of the general formula (IV) taken alone is another embodiment of the instant invention.

### DETAILED DESCRIPTION OF THE INVENTION

The present invention provides a process for producing carbonyl isothiocyanates and their derivatives. In particular, the present process is used to produce N-alk(en)oxy(or aryloxy)carbonyl isothiocyanate derivatives in high yield and purity. The process comprises reacting a haloformate compound of the general formula (I) 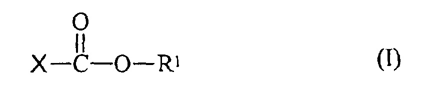 wherein R¹ represents a C₁ - C₈ alkyl radical, a C₂ - C₄ alkenyl radical or a C₆ - C₁₀ aryl radical; and X represents a halogen atom,
with a thiocyanate of the general formula (II)

MSCN (II)

wherein M represents an alkali or alkaline earth metal, lead, or NH₄,
in the presence of an aqueous solvent, and in the presence of a catalytic amount of a N,N-dialk(en)ylarylamine of the general formula (III) 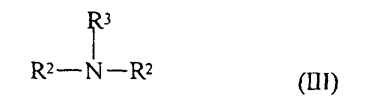 wherein the two R² each independently of another represent a C₁ - C₈ alkyl radical or a C₃ - C₆ alkenyl radical, or the two R² together represent a C₅ saturated heterocyclic ring or a C₄ saturated heterocyclic ring wherein an oxygen atom may be part of the ring; and R³ represents an aryl group that can be a phenyl, a naphthyl, a substituted phenyl or a substituted naphthyl; to produce a N-alk(en)oxy(or aryloxy)carbonyl isothiocyanate intermediate product of the general formula (IV) 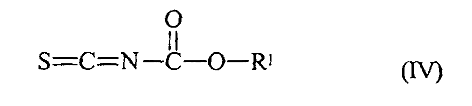 wherein R¹ is as defined above in formula (I); and reacting a compound of the general formula (V)

R⁴ - Y - H (V)

wherein R⁴ represents a C₁ - C₁₀ alkyl radical, a C₆ - C₁₀ aryl radical or a C₁ - C₈ alkoxy radical, and Y represents oxygen, sulfur or NR⁵, wherein R⁵ represents hydrogen or R⁴, with the intermediate product (IV) to produce a N-alkoxy(or aryloxy)carbonyl isothiocyanate derivative of the general formula (VI) 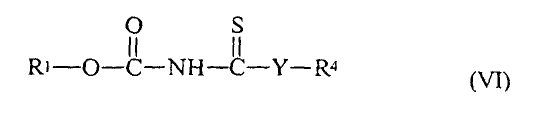 wherein R¹ is as defined above in formula (I), and R⁴ and Y are as defined above in formula (V).

In another embodiment of the invention the process comprises reacting a haloformate compound of the general formula (I) 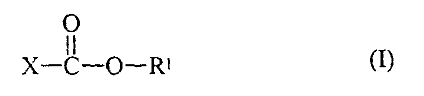 wherein R¹ represents a C₁ - C₈ alkyl radical, a C₂ - C₄ alkenyl radical or a C₆ -C₁₀ aryl radical; and X represents a halogen atom, with a thiocyanate of the general formula (II)

MSCN (II)

wherein M represents an alkali or alkaline earth metal, lead, or NH₄,
in the presence of an organic solvent, and in the presence of a catalytic amount of a N,N-dialk(en)ylarylamine of the general formula (III) 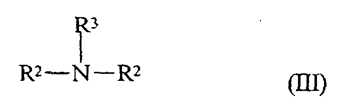 wherein the two R² each independently of another represent a C₁ - C₈ alkyl radical or a C₃ - C₆ alkenyl radical, or the two R² together represent a C₅ saturated heterocyclic ring or a C₄ saturated heterocyclic ring wherein an oxygen atom may be part of the ring; and R³ represents an aryl group that can be a phenyl, a naphthyl, a substituted phenyl or a substituted naphthyl; to produce a N-alk(en)oxy(or aryloxy)carbonyl isothiocyanate intermediate product of the general formula (IV) 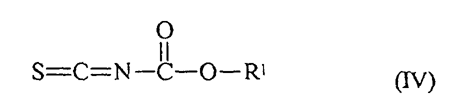 wherein R¹ is as defined above in formula (I); and reacting a compound of the general fonnula (V)

R⁴ - Y - H (V)

wherein R⁴ represents a C₁ - C₁₀ alkyl radical, a C₆ - C₁₀ aryl radical or a C₁ - C₈ alkoxy radical, and Y represents oxygen, sulfur, or NR⁵, wherein R⁵ represents hydrogen or R⁴, with the intermediate product (IV) to produce a N-alkoxy(or aryloxy)carbonyl isothiocyanate derivative of the general formula (VI) 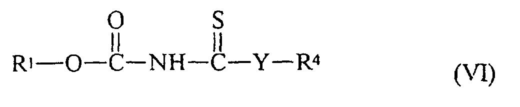 wherein R¹ is as defined above in formula (I), and R⁴ and Y are as defined above in formula (V).

The process of the present invention may be conducted in a one-pot process.

The process according to the invention is generally carried out at atmospheric pressure.

In the embodiment of the invention, wherein an aqueous solvent is used in the first reaction step (reaction of the haloformate of general formula (I) with the thiocyanate of general formula (II)), this reaction step is carried out at a temperature of from about -10°C to about 40°C; and preferably at a temperature of from about 0°C to about 10°C.

In the embodiment of the invention, wherein an organic solvent is used in the first reaction step (reaction of the haloformate (formula (I)) with the thiocyanate (formula (II)), this reaction step is carried out at a temperature of from about -10°C to about 116°C; and preferably at a temperature of from about 20°C to about 40°C.

The haloformate is added to the reaction mixture at a rate such that the temperature of the reaction remains in the desired range. The reaction time for this step in the process of the invention is up to about 16 hours; and preferably the reaction time is from about 1 hour to about 4 hours. The progress of the reaction is monitored by liquid chromatography analysis of the reaction mixture to determine the amount of unreacted thiocyanate.

Suitable haloformates for use in the process of the present invention include the following chloroformates: methyl, ethyl, propyl, isopropyl, butyl, isobutyl, amyl, hexyl, 2-ethylhexyl, benzyl, phenyl, and allyl chloroformates. In a preferred embodiment, the haloformate is either methyl chloroformate or propyl chloroformate.

Suitable thiocyanates for use in the process of the present invention include metal, lead and ammonium thiocyanates. Suitable thiocyanates include sodium, lithium, potassium, rubidium, cesium, beryllium, magnesium, calcium, strontium, and barium thiocyanates. In a preferred embodiment, the thiocyanate is sodium thiocyanate.

The reaction of the haloformate and thiocyanate is carried out in the presence of a catalytic amount of a N,N-dialk(en)ylarylamine (formula III), and in the presence of an aqueous solvent or of an organic solvent.

Suitable N,N-dialk(en)ylarylamines for use as a catalyst in the reaction of the present invention include N,N-dimethylaniline, N,N-dimethyl-1-naphthylamine, N,N-dimethyl-p-toluidine, N,N-diethylaniline, N,N-diallylaniline, 1-phenylpiperidine and 4-phenylmorpholine. In a preferred embodiment, the N,N-dialkyl(en)arylamine catalyst is N,N-dimethylaniline. The amount of catalyst present in the reaction mixture is such that it comprises from about 0.1% to about 30% by mole based on the haloformate; and preferably from about 3% to about 9% by mole.

Depending on the choice of the embodiment of the instant invention suitable solvents for use in the process of the present invention include either aqueous solvents such as water or include organic solvents. As such suitable organic solvents there may be mentioned alcohols such as methanol, ethanol, propanol, butanol; nitriles such as acetonitrile, propionitrile or butyronitrile; aromatic compounds such as benzene, toluene, xylene; halogenated hydrocarbons such as carbon tetrachloride; tetrahydrofuran; and ketones such as acetone or methyl isobutyl ketone. In a preferred embodiment, the solvent is methyl isobutyl ketone.

If an aqueous solvent is used, then there may be mentioned another embodiment of the present invention, wherein, following completion of the reaction between the haloformate and the thiocyanate, an acid such as aqueous hydrochloric acid or aqueous sulfuric acid may be added to the reaction mixture to neutralize the catalyst.

In the embodiment of the invention, wherein an aqueous solvent is used, the reaction of the N-alk(en)oxy(or aryloxy)carbonyl isothiocyanate intermediate product of formula (IV) with the compound of formula (V) is carried out at a temperature of from about -10°C to about 100°C, and preferably from about 25°C to about 50°C; for a time period of up to about 16 hours, and preferably from about 2 hours to about 4 hours.

In the embodiment of the invention, wherein an organic solvent is used, the reaction of the N-alk(en)oxy(or aryloxy)carbonyl isothiocyanate intermediate product of formula (IV) with the compound of formula (V) is carried out at a temperature of from about -10°C to about 116°C, and preferably from about 25°C to about 50°C, for a time period of up to about 16 hours, and preferably from about 2 hours to about 4 hours.

Suitable compounds represented by formula (V) for use in the process of the present invention include alcohols such as methanol, ethanol, n-propyl alcohol, isopropyl alcohol, n-butyl alcohol, isobutyl alcohol, sec-butyl alcohol, amyl alcohols, hexyl alcohols, heptyl alcohols, cyclopentyl alcohol, cyclohexyl alcohol, allyl alcohols, benzyl alcohol; amines such as methylamine, ethylamine, hexylamine, isopropylaminc, isobutylamine, amylamines, cyclohexylamine, octylamine, benzylamine, dimethylamine, diethylamine, dipropylamine, dibutylamine, diphenylamine, dibenzylamine, ethylmethylamine, N-methylaniline; mercaptans such as methyl mercaptan, ethyl mercaptan, propyl mercaptan, butyl mercaptan, amyl mercaptans, hexyl mercaptans, benzyl mercaptans, allyl mercaptans and the like. Preferred compounds of formula (V) are methanol and propanol.

If an organic solvent is used, then there may be mentioned another embodiment of the present invention, wherein, following completion of the reaction between the N-alk(en)oxy(or aryloxy)carbonyl isothiocyanate intermediate product and the compound of formula (V), an acid such as aqueous hydrochloric acid or aqueous sulfuric acid may be added to the reaction mixture to neutralize the catalyst.

The reaction step, which comprises reacting a haloformate compound of the general formula (I) 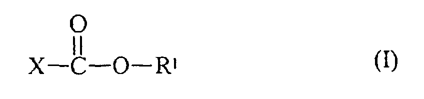 wherein R¹ represents a C₁ - Cg alkyl radical, a C₂ - C₄ alkenyl radical or a C₆ -C₁₀ aryl radical; and X represents a halogen atom, with a thiocyanate of the general formula (II)

MSCN (II)

wherein M represents an alkali or alkaline earth metal, lead, or NH₄,
in the presence of an aqueous or an organic solvent, and in the presence of a catalytic amount of a N,N-dialk(en)ylarylamine of the general formula (III) 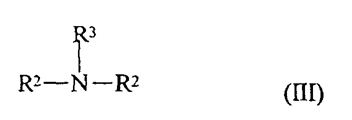 wherein the two R² each independently of another represent a C₁ - C₈ alkyl radical or a C₃ - C₆ alkenyl radical, or the two R² together represent a C₅ saturated heterocyclic ring or a C₄ saturated heterocyclic ring wherein an oxygen atom may be part of the ring; and R³ represents an aryl group that can be a phenyl, a naphthyl, a substituted phenyl or a substituted naphthyl; to produce a N-alk(en)oxy(or aryloxy)carbonyl isothiocyanate intermediate product of the general formula (IV) 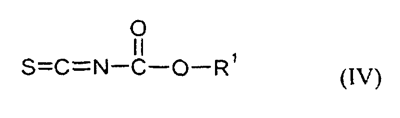 wherein R¹ is as defined above in formula (I) is itself another embodiment of the instant invention.

The invention is further illustrated but is not intended to be limited by the following examples in which all parts and percentages are by weight unless otherwise specified.

### EXAMPLES

### Example 1 - Preparation of MTC, With Neutralization of the Catalyst

7.2 grams (0.06 mole) of 99% pure N,N-dimethylaniline was added to a solution of 86.0 grams (1.04 moles) of 98% pure NaSCN, in 170.5 grams of water, and the mixture was cooled to a temperature of from about 0°C to about 5°C with stirring. About 95.5 grams (1.00 mole) of 99% pure methyl chloroformate was added to the stirred reaction mixture over a period of about 2 hours, maintaining the temperature of the reaction mixture from about 0°C to about 5°C. Following this addition step, the reaction mixture was stirred for about 1 hour. The reaction mixture was then chilled to a temperature of about 0°C. A chilled (about 0°C) solution of 6.18 grams (0.06 mole) of sulfuric acid was dissolved in 174 ml of water, and this solution was added to the reaction mixture (to neutralize the catalyst) over a time period of about 10 minutes. The mixture was then stirred at a temperature of about 0°C for about 15 minutes. The stirring was then stopped and the mixture was allowed to settle for about 15 minutes at a temperature of about 0°C. The mixture separated into an aqueous phase and an organic phase. The desired N-methoxycarbonyl isothiocyanate product ("MITC") was contained within the organic phase.

While maintaining the reaction mixture at a temperature of about 0°C, the lower organic layer (which included the intermediate MITC product) was slowly added to about 277.6 grams (8.68 moles) of methanol, over a time period of about 1 hour, while the temperature of the reaction mixture was maintained between about 30°C and about 40°C. The reaction mixture was then stirred at a temperature of from about 30°C to about 40°C for about 2 hours. The solvent-free purity of N-methoxycarbonyl-O-methylthionocarbamate ("MTC") in the reaction mixture at this point was 98.5% (no trace of the isomer MeOOC-SCN was observed using a liquid chromatograph).

Analysis by liquid chromatography of the resulting solution of MTC in methanol resulted in a yield of 93.4% based on methyl chloroformate.

### Example 2 - Preparation of MTC; Without Neutralizing the Catalyst

22.0 grams (0.18 mole) of 99% pure N,N-dimethylaniline was added to a solution of 248.3 grams (3.00 moles) of 98% pure NaSCN, in 595.0 grams of water. The mixture was cooled to a temperature of between about 0°C and about 5°C with stirring. About 274.9 grams (2.88 moles) of 99% pure methyl chloroformate was added to the stirred reaction mixture over a time period of about 2 hours, maintaining the temperature of the reaction mixture between about 0°C and about 5°C. Following addition of the methyl chloroformate, the reaction mixture was then stirred at a temperature of between about 0°C and about 5°C for a period of about 1 hour. The stirring was then stopped and the phases were allowed to separate into an organic and an aqueous phase at a temperature of about 0°C, without prior neutralization of the catalyst. The organic layer (containing the intermediate "MITC" product) was slowly added to 800.0 grams (25.0 moles) of methanol over a period of about 1 hour, while the temperature of the reaction mixture was maintained between about 30°C and about 40°C. The reaction mixture was then stirred at a temperature of from about 30°C to about 40°C for about 2 hours.

Analysis by liquid chromatography of the resulting solution of N-methoxycarbonyl-O-methylthionocarbamate ("MTC") in methanol indicated a yield of 93% based on methyl chloroformate.

### Example 3 - Preparation of PTC; Without Neutralizing the Catalyst

22.0 grams (0.18 mole) of 99% pure N,N-dimethylaniline was added to a solution of 260.4 grams (3.15 moles) of 98% pure NaSCN, in 640.0 grams of water. The mixture was cooled to a temperature of between about 0°C and about 5°C with stirring. 376.8 grams (3.01 moles) of 98% pure propyl chloroformate was added to the stirred reaction mixture over a period of about 2 hours, maintaining the temperature of the reaction mixture between about 0°C and about 5°C. Following addition of the propyl chloroformate, the reaction mixture was stirred at a temperature of between about 0°C and about 5°C for a period of about 4 hours. The stirring was then stopped and the phases were allowed to separate into an organic phase and an aqueous phase at a temperature of about 0°C, without prior neutralization of the catalyst. The desired N-propoxycarbonyl isothiocyanate product ("PITC") was contained within the organic phase. The organic layer (containing the intermediate PITC product) was slowly added to 540.0 grams (9.0 moles) of propanol over a period of about 1 hour. During this addition step, the temperature of the organic layer was maintained at about 0°C; while the temperature of the resultant reaction mixture was maintained between about 30°C and about 40°C. The reaction mixture was then stirred at a temperature of from about 30°C to about 40°C for about 2 hours.

Analysis by liquid chromatography of the resulting solution of N-propoxycarbonyl-O-propylthionocarbamate ("PTC") in propanol indicated a yield of 98% based on propyl chloroformate.

### Example 4

8.3 grams (0.1 mole) of 98% pure sodium thiocyanate (NaSCN), 0.37 grams (0.003 mole) of the catalyst N,N-dimethylaniline, and 75 ml of methyl isobutyl ketone (MIBK) were charged to a reactor. The reaction mixture was stirred and heated to a temperature of about 85°C. The reaction mixture was made anhydrous by azeotropic distillation of a small amount of the solvent (about 20 ml) under a reduced pressure of about 200 mm of mercury. The reaction mixture was then cooled to room temperature under a nitrogen atmosphere. About 10.5 grams (0.11 mole) of 99% pure methyl chloroformate was added over a time period of about 30 minutes, maintaining the temperature of the reaction mixture between about 25°C and about 40°C. The reaction mixture was then stirred for about 4 hours at room temperature to complete the formation of the intermediate N-methoxycarbonyl isothiocyanate ("MITC").

To convert the MITC intermediate product to N-methoxycarbonyl-O-methyl thionocarbamate ("MTC"), 6.4 grams (0.20 mole) of methanol was added to the reaction mixture over a time period of about 15 minutes at room temperature. The mixture was then stirred at a temperature of about 50°C for a time period of about 4 hours. The reaction mixture was then cooled to room temperature and the catalyst was neutralized by addition of a mixture of 30 ml of water and 3 ml of concentrated hydrochloric acid. The mixture was stirred for a period of about 15 minutes. The stirring was stopped, the mixture was allowed to settle, and the mixture separated into an organic phase and an aqueous phase. The organic phase (which contained the MTC product) was dried over anhydrous magnesium sulfate and the solvent was removed using a rotary evaporator at a temperature of about 50°C to obtain 13.82 grams of crude MTC as residue. The purity of crude MTC was 98.9% (using liquid chromatography), which translated to a net yield of 91.7% based on the sodium thiocyanate.

## Claims

1. A process for the preparation of N-alk(en)oxy(or aryloxy)carbonyl isothiocyanate derivatives comprising:
a) reacting a haloformate compound of the general formula (I) 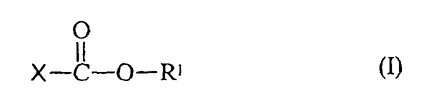 wherein R¹ represents a C₁ - C₈ alkyl radical, a C₂ - C₄ alkenyl radical, or a C₆ - C₁₀ aryl radical, and X represents a halogen atom, with a thiocyanate of the general formula (II)
MSCN (II)
wherein M represents an alkali or alkaline earth metal, lead, or NH₄, in the presence of an aqueous solvent or an organic solvent, and in the presence of a catalytic amount of a N,N-dialk(en)ylarylamine of the general formula (III) 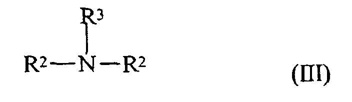 wherein the two R² each independently of another represent a C₁ - C₈ alkyl radical or a C₃ - C₆ alkenyl radical, or the two R² together represent a C₅ saturated heterocyclic ring or a C₄ saturated heterocyclic ring wherein an oxygen atom may be part of the ring; and R³ represents an aryl group that can be a phenyl, a naphthyl, a substituted phenyl or a substituted naphthyl,
to produce a N-alk(en)oxy (or aryloxy)carbonyl isothiocyanate intermediate product of the general formula (IV) 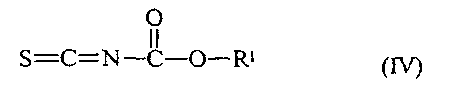 wherein R¹ is as defined above in formula (I); and
b) reacting the intermediate product (IV) with a compound of the general formula (V)
R⁴ - Y - H (V)
wherein R⁴ represents a C₁ - C₁₀ alkyl radical, a C₆ - C₁₀ aryl radical or a C₁ - C₈ alkoxy radical, and Y represents oxygen, sulfur, or NR⁵, wherein R⁵ represents hydrogen or R⁴, to produce a N-alk(en)oxy(or aryloxy)carbonyl isothiocyanate derivative of the general formula (VI) 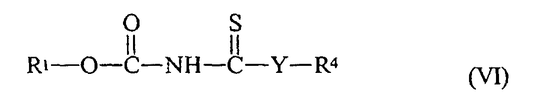 wherein R¹ is as defined above in formula (I), and R⁴ and Y are as defined above in formula (V).

2. The process of Claim 1 wherein the haloformate compound is selected from the group of chloroformates consisting of methyl, ethyl, propyl, isopropyl, butyl, isobutyl, amyl, hexyl, 2-ethylhexyl, benzyl, phenyl, and allyl chloroformate.

3. The process of Claim 1 wherein in step a) the reaction is carried out in the presence of an aqueous solvent at a temperature of from -10°C to 40°C and in step b) the reaction is carried out at a temperature of from -10°C to 100°C.

4. The process of Claim 1, wherein in step a) the reaction is carried out in the presence of an organic solvent at a temperature of from -10°C to 116°C and in step b) the reaction is carried out at a temperature of from -10°C to 116°C.

5. The process of Claim 1 wherein the N,N-dialk(en)ylarylamine is selected from the group consisting of N,N-dimethylaniline, N,N-dimethyl-1-naphthylamine, N,N-dimethyl-p-toluidine, N,N-diethylaniline, N,N-diallylaniline, 1-phenylpiperidine, and 4-phenylmorpholine.

6. The process of Claim 1 wherein the N,N-dialk(en)ylarylamine comprises from 0.1% to 30% by mole based on the haloformate.

7. The process of Claim 1 wherein the aqueous solvent is water.

8. The process of Claim 1 wherein step a) is carried out in the presence of an aqueous solvent and this step a) further comprises the addition of an acid to the reaction mixture to neutralize the catalyst.

9. The process of Claim 1 wherein step a) is carried out in the presence of an organic solvent and step b) further comprises the addition of an acid to the reaction mixture to neutralize the catalyst.

10. A process for the preparation of N-alk(en)oxy(or aryloxy)carbonyl isothiocyanates comprising reacting a haloformate compound of the general formula (I) 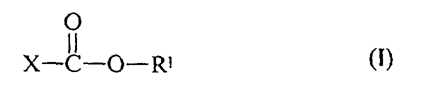 wherein
R¹ represents a C₁ - C₈ alkyl radical, a C₂ - C₄ alkenyl radical or a C₆ -C₁₀. aryl radical; and X represents a halogen atom, with a thiocyanate of the general formula (II)
MSCN (II)
wherein
M represents an alkali or alkaline earth metal, lead, or NH₄, in the presence of an aqueous or organic solvent, and in the presence of a catalytic amount of a N,N-dialk(en)ylarylamine of the general formula (III) 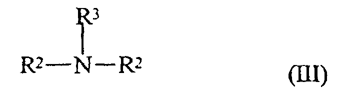 wherein
the two R² each independently of another represent a C₁ - C₈ alkyl radical or a C₃ - C₆ alkenyl radical, or the two R² together represent a C₅ saturated heterocyclic ring or a C₄ saturated heterocyclic ring wherein an oxygen atom may be part of the ring; and R³ represents an aryl group that can be a phenyl, a naphthyl, a substituted phenyl or a substituted naphthyl; to produce a N-alk(en)oxy (or aryloxy)carbonyl isothiocyanate intermediate product of the general formula (IV) 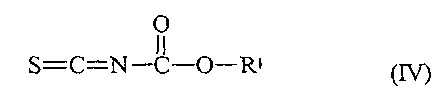 wherein
R¹ is as defined above in formula (I).

## Patentansprüche

1. Verfahren zur Herstellung von N-Alk(en)oxy- oder N-Aryloxycarbonylisothiocyanat-Derivaten, umfassend:
a) das Umsetzen einer Halogenameisensäureester-Verbindung der allgemeinen Formel (I) 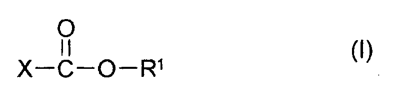 worin R¹ für einen C₁-C₈-Alkylrest, einen C₂-C₄-Alkenylrest oder einen C₆-C₁₀-Arylrest steht und X für ein Halogenatom steht, mit einem Thiocyanat der allgemeinen Formel (II)
MSCN (II)
worin M für ein Alkali- oder Erdalkalimetall, Blei oder NH₄ steht, in Gegenwart eines wässrigen Lösungsmittels oder eines organischen Lösungsmittels und in Gegenwart einer katalytischen Menge eines N,N-Dialk(en)ylarylamins der allgemeinen Formel (III) 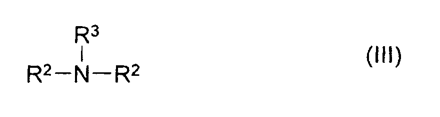 worin die beiden R² jeweils unabhängig voneinander für einen C₁-C₈-Alkylrest oder einen C₃-C₆-Alkenylrest stehen oder die beiden R² zusammen einen gesättigten heterozyklischen C₅-Ring oder einen gesättigten heterozyklischen C₄-Ring bilden, worin gegebenenfalls ein Sauerstoffatom Teil des Rings ist; und R³ für eine Arylgruppe steht, die Phenyl, Naphthyl, substituiertes Phenyl oder substituiertes Naphthyl sein kann, um ein N-Alk(en)oxy- oder N-Aryloxycarbonylisothiocyanat-Zwischenprodukt der allgemeinen Formel (IV) herzustellen 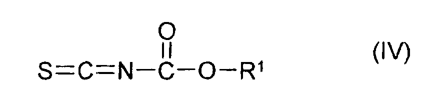 worin R¹ wie oben in Formel (I) definiert ist; und
b) das Umsetzen des Zwischenprodukts (IV) mit einer Verbindung der allgemeinen Formel (V)
R⁴-Y- H (V)
worin R⁴ für einen C₁-C₁₀-Alkylrest, einen C₆-C₁₀-Arylrest oder einen C₁-C₈-Alkoxyrest steht und Y für Sauerstoff, Schwefel oder NR⁵ steht, worin R⁵ für Wasserstoff oder R⁴ steht,
um ein N-Alk(en)oxy- oder N-Aryloxycarbonylisothiocyanat-Derivat der allgemeinen Formel (VI) herzustellen 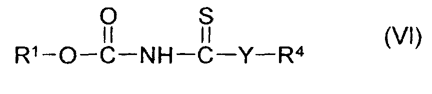 worin R¹ wie oben in Formel (I) definiert ist und R⁴ und Y wie oben in Formel (V) definiert sind.

2. Verfahren nach Anspruch 1, worin die Halogenameisensäureester-Verbindung aus der aus Chlorameisensäuremethyl-, -ethyl-, -propyl-, -isopropyl-, -butyl-, -isobutyl-, -amyl-, -hexyl-, -2-ethylhexyl-, -benzyl-, -phenyl- und -allylester bestehenden Gruppe von Chlorameisensäureester-Verbindungen ausgewählt ist.

3. Verfahren nach Anspruch 1, worin die Umsetzung in Schritt a) in Gegenwart eines wässrigen Lösungsmittels und bei einer Temperatur von -10 °C bis 40 °C und die Umsetzung in Schritt (b) bei einer Temperatur von -10 °C bis 100 °C durchgeführt wird.

4. Verfahren nach Anspruch 1, worin die Umsetzung in Schritt a) in Gegenwart eines organischen Lösungsmittels bei einer Temperatur von -10 °C bis 116 °C und die Umsetzung in Schritt b) bei einer Temperatur von -10 °C bis 116 °C durchgeführt wird.

5. Verfahren nach Anspruch 1, worin das N,N-Dialk(en)ylarylamin aus der aus N,N-Dimethylanilin, N,N-Dimethyl-1-naphthylamin, N,N-Dimethyl-p-toluidin, N,N-Diethylanilin, N,N-Diallylanilin, 1-Phenylpiperidin und 4-Phenylmorpholin bestehenden Gruppe ausgewählt ist.

6. Verfahren nach Anspruch 1, worin das N,N-Dialk(en)ylarylamin in einer Menge von 0,1 Mol-% bis 30 Mol-% des Halogenameisensäureesters vorliegt.

7. Verfahren nach Anspruch 1, worin das wässrige Lösungsmittel Wasser ist.

8. Verfahren nach Anspruch 1, worin Schritt a) in Gegenwart eines wässrigen Lösungsmittels durchgeführt wird und dieser Schritt a) außerdem den Zusatz einer Säure zum Reaktionsgemisch umfasst, um den Katalysator zu neutralisieren.

9. Verfahren nach Anspruch 1, worin Schritt a) in Gegenwart eines organischen Lösungsmittels durchgeführt wird und Schritt b) außerdem den Zusatz einer Säure zum Reaktionsgemisch umfasst, um den Katalysator zu neutralisieren.

10. Verfahren zur Herstellung von N-Alk(en)oxy- oder N-Aryloxycarbonylisothiocyanaten, umfassend das Umsetzen einer Halogenameisensäureester-Verbindung der allgemeinen Formel (I) 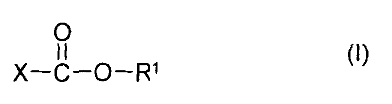 worin R¹ für einen C₁-C₈-Alkylrest, einen C₂-C₄-Alkenylrest oder einen C₆-C₁₀-Arylrest steht und X für ein Halogenatom steht, mit einem Thiocyanat der allgemeinen Formel (II)
MSCN (II)
worin M für ein Alkali- oder Erdalkalimetall, Blei oder NH₄ steht, in Gegenwart eines wässrigen Lösungsmittels oder eines organischen Lösungsmittels und in Gegenwart einer katalytischen Menge eines N,N-Dialk(en)ylarylamins der allgemeinen Formel (III) 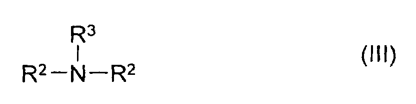 worin die beiden R² jeweils unabhängig voneinander für einen C₁-C₈-Alkylrest oder einen C₃-C₆-Alkenylrest stehen oder die beiden R² zusammen einen gesättigten heterozyklischen C₅-Ring oder einen gesättigten heterozyklischen C₄-Ring stehen, worin gegebenenfalls ein Sauerstoffatom Teil des Rings ist; und R³ für eine Arylgruppe steht, die Phenyl, Naphthyl, substituiertes Phenyl oder substituiertes Naphthyl sein kann;
um ein N-Alk(en)oxy- oder N-Aryloxycarbonylisothiocyanat-Zwischenprodukt der allgemeinen Formel (IV) herzustellen 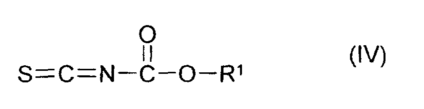 worin R¹ wie oben in Formel (I) definiert ist.

## Revendications

1. Procédé pour la préparation de dérivés de N-alkyl(ène)oxy- ou N-aryloxy-carbonyl-isothiocyanate qui comprend :
a) la réaction d'un composé d'haloformiate de formule générale (I) 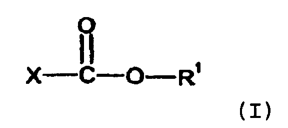 dans laquelle R¹ représente un radical alkyle en C₁-C₈, un radical alcényle en C₂-C₄ ou un radical aryle en C₆-C₁₀; et X représente un atome d'halogène, avec un thiocyanate de formule générale (II)
**MSCN** (II)
dans laquelle M représente un métal alcalin ou alcalino-terreux, le plomb ou NH₄,
en présence d'un solvant aqueux ou d'un solvant organique, et en présence d'une quantité catalytique d'une N,N-dialkyl(ène)arylamine de formule générale (III) 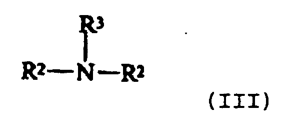 dans laquelle les deux R² représentent chacun indépendamment l'un de l'autre un radical alkyle en C₁-C₈ ou un radical alcényle en C₃-C₆ ou les deux R² représentent conjointement un hétérocycle saturé en C₅ ou un hétérocycle saturé en C₄ où un atome d'oxygène peut être une partie du cycle; et R³ représente un groupement aryle qui peut être un phényle, un naphtyle, un phényle substitué ou un naphtyle substitué;
pour produire un produit intermédiaire de N-alkyl(ène)oxy- ou N-aryloxy-carbonyl-isothiocyanate de formule générale (IV) 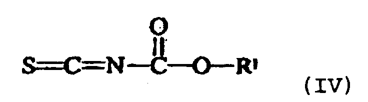 dans laquelle R¹ est comme défini ci-dessus dans la formule (I); et
b) la réaction d'un produit intermédiaire (IV) avec un composé de formule générale (V)
**R**^{**4**} **- Y - H** (V)
dans laquelle R représente un radical alkyle en C₁-C₁₀, un radical aryle en C₆-C₁₀ ou un radical alkoxy en C₁-C₈ et Y représente l'oxygène, le soufre ou NR⁵, où R⁵ représente hydrogène ou R⁴, pour produire un dérivé de N-alkyl(ène)oxy- ou N-aryloxy-carbonyl-isothiocyanate de formule générale (VI) 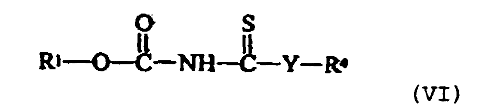 dans laquelle R¹ est comme défini ci-dessus dans la formule (I), et R⁴ et Y sont comme définis ci-dessus dans la formule (V).

2. Procédé selon la revendication 1 dans lequel le composé d'haloformiate est choisi parmi le groupe des chloroformiates constitué des chloroformiates de méthyle, d'éthyle, de propyle, d'isopropyle, de butyle, d'isobutyle, d'amyle, d'hexyle, de 2-éthylhexyle, de benzyle, de phényle et d'allyle.

3. Procédé selon la revendication 1 dans lequel dans l'étape a) la réaction est effectuée en présence d'un solvant aqueux à une température de -10°C à 40°C et dans l'étape (b) la réaction est effectuée à une température de -10°C à 100°C.

4. Procédé selon la revendication 1 dans lequel dans l'étape a) la réaction est effectuée en présence d'un solvant organique à une température de -10°C à 116°C et dans l'étape (b) la réaction est effectuée à une température de -10°C à 116°C.

5. Procédé selon la revendication 1 dans lequel la N,N-dialkyl(ène)arylamine est choisie parmi le groupe constitué de la N,N-diméthylaniline, la N,N-diméthyl-1-naphtylamine, la N,N-diméthyl-p-toluidine, la N,N-diéthylamine, la N,N-diallylamine, la 1-phénylpipéridine et la 4-phénylmorpholine.

6. Procédé selon la revendication 1 dans lequel la N,N-dialkyl(ène)arylamine comprend 0,1% à 30% en mole sur base de l'haloformiate.

7. Procédé selon la revendication 1 dans lequel le solvant aqueux est l'eau.

8. Procédé selon la revendication 1 dans lequel l'étape a) est effectuée en présence d'un solvant aqueux et cette étape a) comprend en outre l'addition d'un acide au mélange de réaction pour neutraliser le catalyseur.

9. Procédé selon la revendication 1 dans lequel l'étape a) est effectuée en présence d'un solvant organique et l'étape b) comprend en outre l'addition d'un acide au mélange de réaction pour neutraliser le catalyseur.

10. Procédé pour la préparation de N-alkyl(ène)oxy- ou N-aryloxy-carbonyl-isothiocyanates comprenant la réaction d'un composé d'haloformiate de formule générale (I) 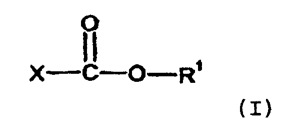 dans laquelle R¹ représente un radical alkyle en C₁-C₈, un radical alcényle en C₂-C₄ ou un radical aryle en C₆-C₁₀; et X représente un atome d'halogène, avec un thiocyanate de formule générale (II)
**MSCN** (II)
dans laquelle M représente un métal alcalin ou alcalino-terreux, le plomb ou NH₄,
en présence d'un solvant aqueux ou organique, et en présence d'une quantité catalytique d'une N,N-dialkyl(ène)arylamine de formule générale (III) 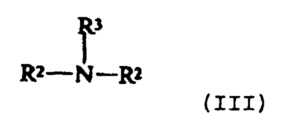 dans laquelle les deux R² représentent chacun indépendamment l'un de l'autre un radical alkyle en C₁-C₈ ou un radical alcényle en C₃-C₆ ou les deux R² représentent conjointement un hétérocycle saturé en C₅ ou un hétérocycle saturé en C₄ où un atome d'oxygène peut être une partie du cycle; et R³ représente un groupement aryle qui peut être un phényle, un naphtyle, un phényle substitué ou un naphtyle substitué; pour produire un produit intermédiaire de N-alkyl(ène)oxy- ou N-aryloxy-carbonyl-isothiocyanate de formule générale (IV) 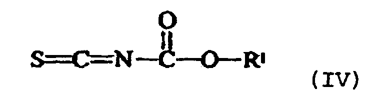 dans laquelle R¹ est comme défini ci-dessus dans la formule (I).
